# EUROPEAN PATENT APPLICATION

(11) **EP 1 270 011 A1**
(43) Date of publication of application: **02.01.2003**
(21) Application number: 01902788.7
(22) Date of filing: 07.02.2001
(51) Int. Cl.: A61K 38/22, A61P 3/08, A61P 3/10, A61P 9/00

(54) **DRUGS FOR AMELIORATING IMPAIRED GLUCOSE TOLERANCE**

(30) Priority: 18.02.2000 JP 2000040585
(71) Applicant: Sumitomo Pharmaceuticals Company, Limited, Osaka-shi, Osaka 541-8510 (JP)
(72) Inventor: KISHINO, Michiko, Suita-shi, Osaka 564-0028 (JP); TAIJI, Mutsuo, Takatsuki-shi, Osaka 569-1044 (JP)
(74) Representative: Cresswell, Thomas Anthony
(86) International application number: JP0100837
(87) International publication number: WO01060398

(57) **Abstract**

Drugs for ameliorating, treating or preventing impaired glucose tolerance, which comprises as the active ingredient a neurotrophic factor such as BDNF or trk receptor agonist.

## Description

### TECHNICAL FIELD

The present invention relates to a drug for ameliorating impaired glucose tolerance, that is a medicament for impaired glucose tolerance to ameliorate, treat or prevent impaired glucose tolerance.

### BACKGROUND ART

In 1980, WHO Expert Committee on Diabetes Mellitus officially approved Impaired Glucose Tolerance (IGT), which had been determined in previous year as one of the classifications of impaired ability of glucose tolerance in the proposal by National Diabetes Data Group of NIH, aiming at the international standardization of names, classification of types of diseases, diagnostic criteria of diabetes mellitus, based on the progress of diabetology. Then, those criteria for judging impaired glucose tolerance were tentatively announced on 1998. According to said judging criteria, the impaired glucose tolerance was defined as events wherein the fasting blood glucose (FPG) is less than 126 mg/dl, and the 2-hour blood glucose level (2hPG) in 75 g oral glucose tolerance test (75 g OGTT) is in the range of from 140 mg/dl to less than 200 mg/dl. The diagnostic criteria of diabetes mellitus by WHO is one exceeding such judging criteria, and is mainly based on the search results on Pima Indians that the 2hPG in 75 g OGTT is at approximate 200 mg/dl which distributes in bimodal, and the frequency of onset of diabetic retinopathy abruptly increases when the 2hPG exceeds this 200 mg/dl level.

The impaired glucose tolerance is a category for classifying persons, whose ability of glucose tolerance becomes exacerbated and becomes lower than the normal level, but does not reach a level to be diagnosed as diabetes mellitus. Persons who are classified as a category of impaired glucose tolerance have a high risk of diabetes mellitus and as well as have a quite high risk of the incidence of arteriosclerosis and cardiovascular diseases. In the recent study, it is found that the macrovascular diseases related to diabetes mellitus precedes that the blood glucose level reaches a level to be diagnosed as diabetes mellitus (Endcr. Rev., 1998). Recently, the impaired glucose tolerance has widely been recognized as a subject to be treated in order to prevent the progress into diabetes mellitus and to prevent the incidence of cardiovascular diseases. In the United States, a large-scale clinical test named Diabetes Prevention Program (DPP) started on patients of impaired glucose tolerance with the support of NIH. The main object of this test is the prevention of the progress into non-insulin-dependent diabetes mellitus and the prevention of the cardiovascular diseases, by treating impaired glucose tolerance.

The impaired glucose tolerance includes the process of development into diabetes mellitus, the improved conditions of diabetes mellitus, the insulin resistant syndrome, and the conditions of a normal person temporarily having a glucose tolerance being exacerbated by a stress, etc. The impaired glucose tolerance is usually found with a higher frequency in fat persons than in non-fat persons, and it is often in association with hyperinsulinemia or insulin resistance with a high probability, and it is apparent that these complications promote the onset of hypertension and hyperlipidemia. In addition, the impaired glucose tolerance may be occurred by a wide variety of causes such as insulin secretion failure in the pancreas, the glucose-uptake failure at the liver, or certain medicaments, many specific hereditary syndromes, or certain diseases optionally accompanied by diabetes mellitus, etc.

Recently, it is pointed out that the number of patients afflicted with diabetes mellitus has been rapidly increased due to aging of population, a change of the dietary life, or lack of exercise owing to the computerization, and the diversification of life style. However, the number of patients who do not show a hyperglycemia to such an extent as diagnosed as diabetes mellitus but show only impaired glucose tolerance is more increased. The persons with impaired glucose tolerance have a high possibility of progressing into diabetes mellitus in future, and have as well a high risk of cardiovascular diseases such as arteriosclerosis, etc. Therefore, it has been desired to develop a drug for ameliorating, treating or preventing impaired glucose tolerance in the medical field.

On the other hand, a neurotrophic factor is a generic name for proteins, which are provided from target cells or neurons and glia cells and Schwann cells in the living body, and show activities to maintain the survival and' differentiation of neurons. They are classified into many types according to the kinds of nerves or receptors to function. Among them, proteins being known as neurotrophins have high structural homology with each other and form a family. The typical examples thereof are neurotrophins such as nerve growth factor (hereinafter, abbreviated as NGF), brain-derived neurotrophic factor (hereinafter, abbreviated as BDNF), neurotrophin 3 (hereinafter, abbreviated as NT-3), neurotrophin 4 (hereinafter, abbreviated as NT-4), neurotrophin 5 (hereinafter, abbreviated as NT-5), or neurotrophin 6 (hereinafter, abbreviated as NT-6); ciliary neurotrophic factor (hereinafter, abbreviated as CNTF); glia cell-derived neurotrophic factor (hereinafter, abbreviated as. GDNF), etc. In addition, neurotrophins are known to act as a specific ligand of receptors (trkA, trkB and/or trkC), which are the products of p75 and trk genes (cf. Takeshi NONOMURA, Hiroshi HATANAKA; Jikken Igaku, vol. 13, p. 376 (1995)).

Neurotrophic factors have been studied with respect to their medical use as a therapeutic agent for treating a patient of neurodegenerative diseases, and its hypoglycemic activity on diabetic animal models has been found. However, neurotrophic factors have not been known to exhibit an ameliorating activity of impaired glucose tolerance on impaired glucose tolerance animal models having a normal non-fasting, blood glucose level.

### DISCLOSURE OF INVENTION

As mentioned above, a drug for ameliorating, treating or preventing impaired glucose tolerance has been desired in the medical field.

When the present inventors administered BDNF, one of neurotrophic factors, to animal models for impaired glucose tolerance exhibiting a normal level of non-fasting blood glucose but exhibiting impaired glucose tolerance, the present inventors have found that neurotrophic factors can (1) not cause hypoglycemia, (2) improve hyperinsulinemia, and (3) maintain the normality of change in the blood glucose level and plasma insulin level in glucose tolerance test for evaluating glucose tolerance, on animal models for impaired glucose tolerance, and have accomplished the present invention.

The present invention relates to a drug for ameliorating impaired glucose tolerance, which comprises as the active ingredient a neurotrophic factor. Moreover, the present invention relates to a drug for ameliorating insulin resistance in patients with impaired of glucose tolerance.

More particularly, the present invention relates to the following:
(1) A drug for ameliorating, treating or preventing impaired glucose tolerance, which comprises as the active ingredient a neurotrophic factor;
(2) A drug for ameliorating insulin resistance in impaired glucose tolerance, which comprises as the active ingredient a neurotrophic factor;
(3) A drug for treating or preventing cardiovascular diseases being caused by impaired glucose tolerance, which comprises as the active ingredient a neurotrophic factor;
(4) A drug for preventing non-insulin-dependent diabetic mellitus being due to impaired glucose tolerance, which comprises as the active ingredient a neurotrophic factor;
(5) A drug according to any one of the above (1) to (4), wherein the impaired glucose tolerance is defined by a fasting blood glucose of less than 126 mg/dl and a 2-hour blood glucose level in 75 g oral glucose tolerance test being in the range of from 140 mg/dl to less than 200 mg/dl;
(6) A drug according to any one of the above (1) to (5), wherein the neurotrophic factor is a trkA, a trkB and/or a trkC receptor agonist.
(7) A drug according to any one of the above (1) to (5), wherein the neurotrophic factor is a brain-derived neurotrophic factor.
(8) A drug according to any one of the above (1) to (5), wherein the neurotrophic factor of the active ingredient is NGF (nerve growth factor), BDNF (brain-derived neurotrophic factor), NT-3 (neurotrophin 3), NT-4 (neurotrophin 4), NT-5 (neurotrophin 5), NT-6 (neurotrophin 6), CNTF (ciliary neurotrophic factor), or GDNF (glia cell-derived neurotrophic factor);
(9) A drug according to any one of the above (1) to (5), wherein the neurotrophic factor of the active ingredient is BDNF (brain-derived neurotrophic factor);
(10) A drug according to any one of the above (1) to (5), wherein the neurotrophic factor of the active ingredient is CNTF (ciliary neurotrophic factor);
(11) A drug according to any one of the above (1) to (5), wherein the neurotrophic factor of the active ingredient is NT-3 (neurotrophin 3);
(12) A drug according to any one of the above (1) to (5), wherein the neurotrophic factor of the active ingredient is NT-4 (neurotrophin 4);
(13) A drug according to any one of the above (1) to (5), wherein the neurotrophic factor of the active ingredient is NT-5 (neurotrophin 5);
(14) A drug according to any one of the above (1) to (5), wherein the neurotrophic factor of the active ingredient is NT-6 (neurotrophin 6);
(15) A drug according to any one of the above (1) to (5), wherein the neurotrophic factor of the active ingredient is NGF (nerve growth factor);
(16) A drug according to any one of the above (1) to (5), wherein the neurotrophic factor of the active ingredient is GDNF (glia cell-derived neurotrophic factor), etc.

### BRIEF DESCRIPTION OF DRAWINGS

Fig. 1 is a graph showing the effects of BDNF administration in the glucose tolerance test on KK mice, and shows the change in blood glucose level with lapse of time.
Fig. 2 is a graph showing the effects of BDNF administration in the glucose tolerance test on KK mice, and shows the change in plasma insulin level with lapse of time.

### BEST MODE FOR CARRYING OUT THE INVENTION

The meaning or definition of each term used in the present specification is explained below.

The "neurotrophic factor" means a physiologically active substance, which is secreted from the target cells for nerve growth, or by autocrine or paracrine, and promotes the growth, differentiation, or survival of neurons to form a neural circuit (synapse) in the living body. For example, the neurotrophic factor includes neurotrophins such as a nerve growth factor (hereinafter, abbreviated as NGF), a brain-derived neurotrophic factor (hereinafter, abbreviated as BDNF), a neurotrophin 3 (hereinafter, abbreviated as NT-3), a neurotrophin 4 (hereinafter, abbreviated as NT-4), a neurotrophin 5 (hereinafter, abbreviated as NT-5), and a neurotrophin 6 (hereinafter, abbreviated as NT-6); ciliary neurotrophic factor (hereinafter, abbreviated as CNTF); glia cell-derived neurotrophic factor (hereinafter, abbreviated as GDNF), etc. In addition, a modified recombinant neurotrophic factor produced by a substitution, a deletion, or an addition of a part of amino acid sequence of the naturally occurred neurotrophic factor sequence by a conventional technique may be included in the neurotrophic factor of the present specification, as far as it exhibits the similar physiological activity.

The. "trkA, trkB and/or trkC receptor agonist" is a generic name . for substances that are bound to trkA, trkB or trkC, which is among the trk gene expression products being known as receptors for "neurotrophin", and activate them to exhibit their activities. Concretely, the known neurotrophin includes, for example, NGF binding to trkA, BDNF and NT-4 binding to trkB, and NT-3 binding to trkC, etc. This concept includes not only modified neurotrophins (modified by amino acid substitution, deletion or addition or sugar chain modification), but also peptides and organic compounds of a lower molecular weight as far as they exhibit a binding ability and activating ability to trkA, trkB or trkC receptor, for example, ability of phosphorylating tyrosine residue.

Among the family of neurotrophic factors, BDNF, NT-3 and NT-4/5 exhibiting a physiological activity through trkB or trkC receptor, which is a trkB or trkC gene expression product, are more useful as a drug for ameliorating, treating or preventing impaired glucose tolerance. Among them, BDNF, which is known to exhibit its physiological activity through trkB receptor, i.e., a trkB gene expression product, is especially preferable as a drug for ameliorating, treating or preventing impaired glucose tolerance.

The impaired glucose tolerance means the following conditions.

The diagnostic classification of glucose metabolism is grouped into diabetic-type (fasting blood glucose level is equal or greater than 126 mg/dl or 2-hour blood glucose level in 75 g oral glucose tolerance test is equal or greater than 200 mg/dl, or the casual blood glucose level is equal or greater than 200 mg/dl), normal-type (fasting blood 'glucose level is less than 110 mg/kg, and 2-hour blood glucose level is less than 140 mg/dl), and borderline-type (not either diabetic-type nor normal-type). The borderline-type is defined as impaired glucose tolerance, and concretely, the impaired glucose tolerance is determined when a fasting blood glucose level is less than 126 mg/dl and a 2-hour blood glucose level in 75 g glucose tolerance test is in the range of from 140 mg/dl to less than 200 mg/dl, which is also called "glucose tolerance disorder". The impaired glucose tolerance scarcely induces complications being characteristic for diabetes mellitus, but has a higher risk of the onset of diabetes mellitus than normal-type, which may be a cause for macrovascular diseases.

The "insulin resistance" means conditions of reduced sensitivity to insulin. Insulin has been known to exhibit a wider range of effects such as, in addition to an effect on the glucose metabolism, an effect on lipid metabolism or effects on the blood vessel and the kidney. Once the sensitivity to insulin is reduced, not only glucose metabolic abnormality but also lipid metabolic abnormality such as hypertriglycemia, decreased HDL plasma level, or hypertension as an abnormality of insulin effects on the blood vessel or the kidney may be induced.

The neurotrophic factors used as the active ingredient of the present invention may be commercially available ones or can be prepared by the following methods.

The neurotrophic factors used as the active ingredient of the present invention can be any ones prepared by various methods as far as they are purified to such a degree that it could be used as a medicament. The neurotrophic factor can be obtained by cultivating a primary culture cell or an established cell line that can produce the neurotrophic factor, and isolating and purifying it from the culture medium thereof (e.g., culture supernatant, cultured cells). Moreover, a recombinant neurotrophic factor can be obtained by a conventional gene engineering technique, e.g., by inserting a gene encoding a neurotrpphic factor into a suitable vector, transforming a suitable host with the recombinant vector, and isolating from a culture supernatant of the resulting transformant. The host cells to be used in the above process are not limited, and may be any conventional host cells which have been used in a gene engineering technique, for example, *Escherichia coli*, *Bacillus subtilis*, yeasts, mold fungi, plant cells or animal cells.

The neurotrophic factors obtained in the above method include a modified recombinant neurotrophic factor such as ones produced by a deletion of a part of amino acid sequence, or a substitution by other amino acid(s), or an addition of a part of other amino acid sequence, or ones having one or more amino acids at the N-terminus and/or the C-terminus, or ones wherein the sugar chain is deleted or substituted, as far as they exhibit substantially the same activity.

### Method for Preparation of BDNF:

When a conventional gene engineering technique is employed, BDNF is prepared by inserting a gene encoding BDNF into a suitable vector, transforming a suitable host with the recombinant vector, and isolating it from a culture supernatant of the resulting transformant (cf., Proc. Natl. Acad. Sci. USA, vol. 88, p. 961 (1991); Biochem. Biophys. Res. Commun., vol. 186, p. 1553 (1992)). The gene engineering technique is suitable for production of BDNF of same quality in a large scale. The host cells mentioned above are not limited, but may be any conventional host cells which have been used in a gene engineering technique, for example, *Escherichia coli*, *Bacillus subtilis*, yeasts, plant cells or animal cells.

### Method for preparation of NT-3:

NT-3 can be prepared by expressing in various host cells in the same manner as in the preparation of BDNF. The methods for preparing thereof and the methods for assay thereof are disclosed in Neuron, vol. 4, 767-773 (1990), or JP-A-5-161493 (WO 91/3659).

### Method for Preparation of Nt-4:

NT-4 can be prepared by expressing in various host cells in the same manner as in the preparation of BDNF. The methods for expression of the recombinant NT-4 and the methods for assay thereof are disclosed in Proc. Natl. Acad. Sci. USA, vol. 89, p. 3060-3064 (1992.4), JP-A-7-509600 (WO 93/25684), or JP-A-6-501617 (WO 92/5254).

### Method for Preparation of CNTF:

CNTF can be prepared in a large scale by expressing in various host cells in the same manner as in the preparation of BDNF. The methods for expression of the recombinant CNTF and the methods for assay thereof are disclosed in Biochimica et Biophysica Acta, vol. 1090, p. 70-80 (1991), J. Neurochemistry, vol. 57, p. 1003-1012 (1991). The methods for expressing the recombinant CNTF and the assay thereof are disclosed in JP-A-4-502916 (WO 90/7341).

The drugs for ameliorating impaired glucose tolerance of the present invention, which comprise as the active ingredient a neurotrophic factor, can be administered either parenterally or orally.

The precise dosage and the administration schedule of the drugs of the present invention should vary according to the dosage to be required for each patient, the method for treatment, the disease to be treated, or the degree of necessity, and further according to the diagnosis by a physician. When administered parenterally, the dosage and the frequency of the administration may vary according to the conditions, ages, body weights of patients, and administration routes, but when it is administered subcutaneously or intravenously in the form of an injection, then the daily dosage thereof is in the range of about 1 to about 2500 µg, preferably in the range of about 10 to about 500 µg, more preferably in the range of about 20 to about 200 µg, per 1 kg of the body weight in an adult. When it is administered to the air tract in the form of an aerosol spray, the daily dosage thereof is in the range of about 1 µg to about 2500 µg, preferably in the range of about 10 to about 500 µg, more preferably in the range of about 20 to about 200 µg, per 1 kg of the body weight in an adult. The administration schedule is. either continuous daily administration, intermittent administration, or a schedule of combining these methods. When administered orally, the dosage and the frequency of administration may vary according to the conditions, ages, body weights of patients, and administration routes, and the daily dosage thereof is in the range of about 5 to about 2500 µg, preferably in the range of about 10 to about 1000 µg per 1 kg of the body weight in an adult.

The exact dosage and the administration schedule of the present drug for ameliorating impaired glucose tolerance should vary according to the dosage to be required for each patient, the method for treatment, kinds of diseases to be treated, or the degree of necessity, and further according to the diagnosis by a physician.

A pharmaceutical composition can be prepared by mixing a neurotrophic factor with a pharmaceutically acceptable non-toxic carrier. When a pharmaceutical composition for parenteral administration (subcutaneous injection, intramuscular injection, or intravenous injection) is prepared, it is preferably in the form of a solution preparation or a suspension preparation. When a pharmaceutical composition for intravaginal administration or rectal administration is prepared, it is preferably in the form of a semi-solid preparation such as cream or suppository. When a pharmaceutical composition for intranasal administration is prepared, it is preferably in the form of a powder, a nasal drop, or an aerosol.

The pharmaceutical composition is administered in the form of a single dosage unit, and can be prepared by any conventional method that is known in the pharmaceutical field such as methods disclosed in Remington's Pharmaceutical Science (published by Mack Publishing Company, Easton, PA, 1970). An injection preparation may optionally contain as a pharmaceutical carrier a protein derived from plasma such as albumin, an amino acid such as glycine, or a carbohydrate such as mannitol, and additionally a buffering agent, a solubilizer, or an isotonic agent, etc. can be contained. When the present pharmaceutical composition is in the form of an aqueous solution preparation or a lyophilized preparation, it may preferably contain a surfactant such as Tween 80 (registered trade mark), Tween 20 (registered trade mark), etc. in order to avoid aggregation. When the present pharmaceutical composition is a composition for parenteral administration other than an injection preparation, then it may contain distilled water or physiological saline solution, polyalkylene glycol such as polyethylene glycol, an oil derived from plant, hydrogenated naphthalene, etc. For example, a pharmaceutical composition such as a suppository for intravaginal administration or rectal administration may contain as a conventional excipient polyalkylene glycol, vaseline, cacao butter, etc. A pharmaceutical composition for intravaginal administration may contain an absorbefacient such as a bile salt, an ethylenediamine salt, a citrate, etc. A pharmaceutical composition for inhalation may be in the form of a solid preparation, and may contain as an excipient lactose, etc., and a pharmaceutical composition for intranasal drop may be in the form of an aqueous solution or an oily solution.

The present pharmaceutical composition is especially preferable in the form of a formulation by which the present drug can persistently be given to a subject by a single administration for a long term, e.g., for one week to one year, and various sustained release preparations, depot preparations, or implant preparations can be employed. For example, a pharmaceutical composition may contain a neurotrophic factor per se, or a pharmaceutically acceptable salt of a neurotrophic factor having a low solubility in body fluid. Such pharmaceutically acceptable salts are, for example, (1): an acid addition salt such as phosphate, sulfate, citrate, tartrate, tannate, pamoate, alginate, polyglutarate, naphthalenemono- or di-sulfonate, polygalacturonate, etc., (2): a salt or complex with polyvalent metal cation such as zinc, calcium, bismuth, barium, nickel, etc, or a combination of (1) and (2), for example, a tannic acid-zinc salt, etc. A neurotrophic factor is preferably converted into a slightly-water-soluble salt thereof, which is mixed with a gel, for example, aluminum monostearate gel arid sesame oil, etc. to give a suitable injection preparation. In this case, especially preferable salt is a zinc salt, a tannic acid-zinc salt, a pamoate, etc. Another type of a sustained release injection preparation is ones wherein a neurotrophic factor is preferably converted into a slightly-water-soluble salt thereof, which is further enclosed in a slow-disintegrative non-toxic and non-antigenic polymer such as a polymer or a copolymer of polylactic acid/polyglycolic acid. In this case, especially preferable salt is zinc salt, tannic acid-zinc salt, pamoate, etc. In addition, a neurotrophic factor or a slightly-water-soluble salt thereof can be enclosed into a cholesterol matrix or a collagen matrix to give a sustained release preparation.

The pharmaceutical preparation for oral administration may be ones which are prepared by microencapsulating a neurotrophic factor or a salt thereof with lecithin, cholesterol, a free fatty acid, or ones which are prepared by enclosing said microcapsules into gelatin capsules, or ones which are prepared by enclosing a neurotrophic factor or a salt thereof in enteric capsules, etc. These preparations may additionally contain,. for example, an absorbefacient, a stabilizer, a surfactant, etc.

### (Toxicity)

When a neurotrophin, especially BDNF, was administered subcutaneously to rats and cynomolgus monkeys at a dose of 100 mg/kg and 60 mg/kg, respectively, for four weeks, no animal died. With respect to the acute toxicity, BDNF was administered to rats and cynomolgus monkeys at a dose of 200 mg/kg or more, and no animal died. Therefore, BDNF shows high safety.

### EXAMPLES

The present invention is illustrated by Examples.

### (1) Materials for experiments:

### Reagents:

BDNF was purchased from REGENERON PHARMACEUTICALS, INC., and used. The other reagents were commercially available ones with the best quality.

### Test animals:

Male KK/Ta mice (SPF standard) were purchased from Clea Japan, Inc. as an animal model for disease. After pre-feeding, the animals were used in the experiment at 12 weeks old.

### Breeding conditions:

The mice were kept in a room being controlled at a temperature of from 20°C to 26°C under a relative humidity of from 30 % to 70%, with an illumination cycle of light on (8:00 to 20:00), and light off (20:00 to 8:00). During the pre-feeding and the experiment, the animals were given food (CE-2, Clea Japan, Inc.) and sterilized tap water *ad libitum.*

### (2) Preparation of dosing solution

As a BDNF solution, Tween 80 and mannitol were dissolved in the stock solution (10 mM phosphoric acid, 0.15 M sodium chloride) to give a test compound solution (10 mM phosphoric acid, 150 mM sodium chloride, 0.01 % Tween 80, 1 % mannitol) containing BDNF at a final concentration of 20 mg/ml.

### (3) Measurement of blood glucose level

The measurement of glucose was carried out in the following manner. The blood (10 µl) was taken from the tail vein of the mice, and mixed with 0.4 N aqueous perchloric acid solution (100 µl). The mixture was neutralized with 0.37 M aqueous potassium carbonate solution (50 µl), and centrifuged. The glucose concentration in the supernatant was measured with Glucose CII Test Wako (a kit for assay of glucose, mutarotase · GOD method, manufactured by Wako Pure Chemical Industries, Ltd.). The absorbance was measured with SPECTRA MAX250 (Micro Plate Reader, manufactured by Molecular Devices Inc.), and calculated by using a software for calculation of concentration being installed in said apparatus, SOFTMAX PRO®, to give the glucose concentration in the whole blood.

### (4) Measurement of plasma insulin level

The insulin level in the plasma was measured by Insulin ELISA kit (Lewis Insulin Mouse, manufactured by SHIBAYAGI CO., LTD.). The absorbance was..measured with SPECTRA MAX250 (Micro Plate Reader, manufactured by Molecular Devices Inc.), and calculated by using a software for calculation of concentration being installed in said apparatus, SOFTMAX PRO®, to give the insulin level. The calculation results. were copied into Macrosoft Excell®, and then the plasma insulin level was calculated by re-multifying with dilution scale.

### (5) Effects of BDNF administration on the non-fasting blood glucose level and non-fasting plasma insulin level of KK mice

KK mice (male, 12 weeks old when administration began) were grouped into three groups (Vehicle-treated group: n=8, BDNF 5 mg/kg-treated group: n=8, and BDNF 20 mg/kg-treated group: n=7) with respect to the blood glucose levels and the body weights thereof, and to each group, the vehicle or BDNF was subcutaneously administered repeatedly daily for 3 weeks. The non-fasting blood glucose level and the plasma insulin level before the administration (Day 0) and on the 18th day (Day 18) were measured in the above-mentioned manner. The results are shown in Table 1.

**Table 1**

| Group of Treated Mice | Measurement date | Blood glucose level (mg/dl) | Plasma insulin level (ng/ml) |
|---|---|---|---|
| Vehicle-treated group (n=8) | Day 0 | 172.8±12.1 | 10.0±5.5 |
| | Day 18 | 192,6±38.1 | 27.2±21.6 |
| BDNF (5 mg/kg)-treated group (n=8) | Day 0 | 177.0±10.1 | 11.3±6.5 |
| | Day 18 | 157.7±9.5* | 12.5±9.0 |
| BDNF (20mg/kg)-treated group (n=7) | Day 0 | 184.0±12.6 | 13.0±8.8 |
| | Day 18 | 149.7±9.6** | 5.2±3.0# |
| The data were expressed as average ± SD, n = 7-8. | | | |

| | | | |
|---|---|---|---|
| * and ** indicate that there is a significant difference of p<0.05, p<0.01 to the vehicle-treated group, respectively, by Williams' test. | | | |
| # indicates that there is a significant difference of p<0.05 to the vehicle-treated group by Dunnet's test. | | | |

As is clear from the above results, the blood glucose levels of the BDNF-treated groups were lower than that of the vehicle-treated group, and they were merely the reduction within normal limits. The plasma insulin levels in the BDNF-treated groups were also significantly lowered as compared to the vehicle-treated group.

### (6) Effects of BDNF on glucose tolerance test in KK mice

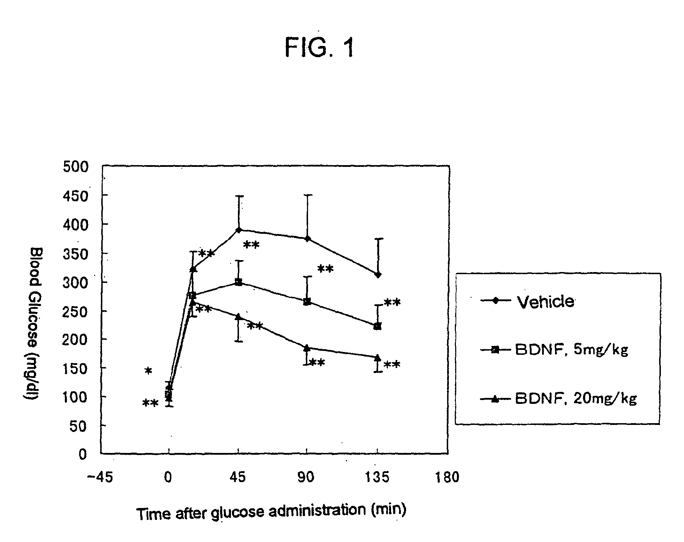

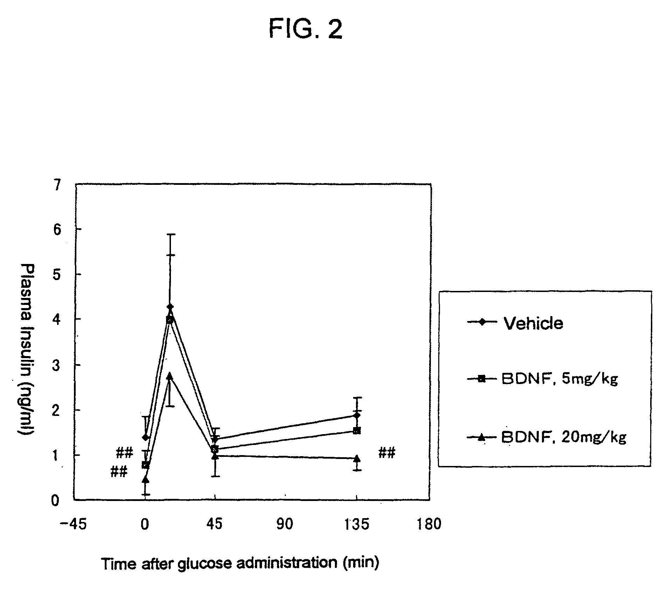

All of the animals to which the vehicle or BDNF had been administered for 3 weeks in the above (5) were fasted overnight, and the glucose tolerance test was carried out on the animals. That is, glucose was orally administered at a dose of 3 g/kg to the mice which had been fasted overnight after the final administration of BDNF, and the blood glucose level and the plasma insulin level before the administration of glucose and on 135 minutes after the administration of glucose were measured, and the changes with time in blood glucose level and in plasma insulin level were studied. The results were indicated in Fig. 1 and Fig. 2, respectively. The data in the drawings were expressed in average ± SD, n=7-8, and ● indicates the data of the vehicle-treated group, ■ indicates the data of the BDNF (5 mg/kg)-treated group, and ▲ indicates the data of the BDNF (20 mg/kg)-treated group. * and ** indicate that there is a significant difference of p<0.05, p<0.01 to the vehicle-treated group, respectively, by Williams' test. ## indicates that there is a significant difference of p<0.01 to the vehicle-treated group by Dunnet's test.

As is shown in Fig. 1 and Fig. 2, both of the blood glucose level and the plasma insulin level in the BDNF-treated groups were undergoing a change at a lower level. From these results, it is apparent that BDNF can ameliorate impaired glucose tolerance and hyperinsulinemia without causing hypoglycemia, and exhibit an activity of improving insulin resistance.

### INDUSTRIAL APPLICABILITY

The drug for ameliorating impaired glucose tolerance of the present invention exhibits an activity of ameliorating glucose tolerance being impaired by various causes, especially impaired glucose tolerance being due to the lack of the insulin activity or insulin resistance. By the administration of the present drug, the development from the impaired glucose tolerance insulin resistant syndrome into various metabolic abnormalities including glucose metabolic abnormalities can be prevented. Therefore, the present drug can be a drug for preventing diabetes mellitus or cardiovascular diseases of a patient with borderline diabetes mellitus, who is not a subject to treatment up to now.

## Claims

1. A drug for ameliorating, treating or preventing impaired glucose tolerance, which comprises as the active ingredient a neurotrophic factor.

2. A drug for ameliorating insulin resistance in impaired glucose tolerance, which comprises as the active ingredient a neurotrophic factor.

3. A drug for ameliorating, treating or preventing cardiovascular diseases being due to impaired glucose tolerance, which comprises as the active ingredient a neurotrophic factor.

4. A drug for preventing non-insulin-dependent diabetes mellitus being due to impaired glucose tolerance, which comprises as the active ingredient a neurotrophic factor.

5. The drug according to any one of claims 1-4, wherein the impaired glucose tolerance is defined as a fasting blood glucose of less than 126 mg/dl, and a 2-hour blood glucose level in 75 g oral glucose tolerance test of from 140 mg/dl to less than 200 mg/dl.

6. The drug according to any one of claims 1 to 5, wherein the neurotrophic factor is a trkA, a trkB and/or a trkC receptor agonist.

7. The drug according to any one of claims 1 to 5, wherein the neurotrophic factor is a brain-derived neurotrophic factor.
